(19) Europäisches Patentamt
European Patent Office
Office européen des brevets

(11) Numéro de publication : **0 459 887 A1**

(12) **DEMANDE DE BREVET EUROPEEN**

(21) Numéro de dépôt : **91401366.9**

(22) Date de dépôt : **28.05.91**

(51) Int. Cl.$^5$ : **C07D 231/14,** A61K 31/415, C07D 231/22, C07D 261/18, C07D 271/10, C07D 285/12, C07D 231/40

(30) Priorité : **30.05.90 FR 9006735**

(43) Date de publication de la demande :
**04.12.91 Bulletin 91/49**

(84) Etats contractants désignés :
**AT BE CH DE DK ES FR GB GR IT LI LU NL SE**

(71) Demandeur : **NOVAPHARME**
**23 rue Jonquoy**
**F-75014 Paris (FR)**

(72) Inventeur : **Lepage, Francis**
**9, allée des Platanes**
**F-60150 Villers Sur Coudun (FR)**
Inventeur : **Hublot, Bernard**
**3, rue des Pommerelles**
**F-60200 Compiègne (FR)**

(74) Mandataire : **Varady, Peter et al**
**Cabinet Lavoix 2, Place d'Estienne d'Orves**
**F-75441 Paris Cedex 09 (FR)**

(54) **Dérivés hétérocycliques doués d'activité anticonvulsivante, procédé de préparation et composition pharmaceutique.**

(57) L'invention a pour objet des composés hétérocycliques doués d'activité anticonvulsivante de formule générale (I)

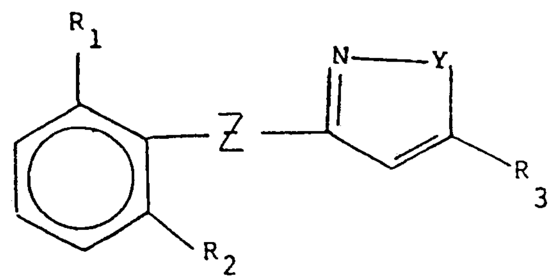

I

dans laquelle
Y est choisi parmi -O-, -S- et

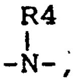

$R_4$ étant H ou un alkyle, acyle ou benzyle éventuellement substitués ;
Z est choisi parmi les groupes -CO-N($R_6$)-, -NH-CO-CH=CH- et-N($R_6$)-CO-, $R_6$ étant H ou alkyle ;
$R_1$ est un alkyle en $C_1$-$C_4$ ; $R_2$ est un alkyle en $C_1$-$C_4$ ou un halogène ; $R_3$ représente un groupe alkyle, alkoxy ou hydroxyalkyle en $C_1$-$C_4$ éventuellement substitués, ou alkanoyle en $C_2$-$C_7$.
Médicaments.

EP 0 459 887 A1

La présente invention se rapporte de manière générale à de nouveaux dérivés hétérocycliques doués d'activité anticonvulsivante, un procédé de préparation ainsi qu'à des compositions thérapeutiques les contenant.

On dispose d'un nombre relativement faible d'agents ayant une activité anticonvulsivante. Parmi ceux-ci un grand nombre présentent des inconvénients liés à des phénomènes d'échappement thérapeutiques, des effets secondaires gênants tels que diminution de la vigilance, somnolence, .. ou des effets toxiques, en particulier hépatotoxiques.

Le but de la présente invention est de fournir de nouveaux composés ayant des propriétés anticonvulsivantes, et exempts des inconvénients de l'art antérieur.

La présente invention a ainsi pour objet des composés hétérocycliques de formule générale

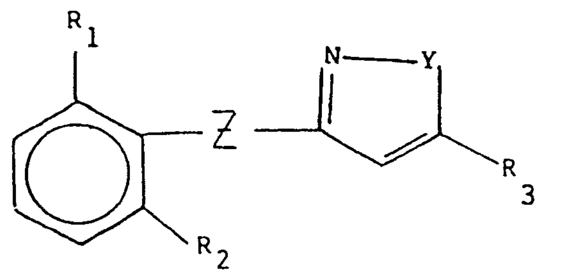

I

dans laquelle

Y est choisi parmi -O-, -S- et

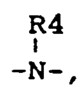

$R_4$ étant choisi parmi un atome d'hydrogène, un groupe alkyle en $C_1$-$C_4$, benzyle, halogénobenzyle, acyle en $C_2$-$C_7$ et un groupe alkyle en $C_1$-$C_4$ substitué par un groupe alkanoyl($C_2$-$C_7$)oxy, dialkyl($C_1$-$C_4$)amino, alkoxy en $C_1$-$C_4$, phénoxy, ou halogénophénoxy,

Z est choisi parmi les groupes -CO-N($R_6$)-, -NH-CO-CH=CH- et-N($R_6$)-CO- dans lesquels $R_6$ est choisi parmi un atome d'hydrogène et un groupe alkyle en $C_1$-$C_4$,

$R_1$ est un groupe alkyle en $C_1$-$C_4$,

$R_2$ est choisi parmi un groupe alkyle en $C_1$-$C_4$, et un atome d'halogène,

$R_3$ est choisi parmi un atome d'hydrogène, un groupe alkyle en $C_1$-$C_4$, alkoxy en $C_1$-$C_4$, hydroxyalkyle en $C_1$-$C_4$, halogénoalkyle en $C_1$-$C_4$, alkanoyle en $C_2$-$C_7$ et un groupe -$CHR_{10}OR_5$ dans lequel $R_5$ est choisi parmi un groupe alkyle en $C_1$-$C_4$, un groupe phényle non substitué ou substitué par un ou plusieurs substituants choisis parmi un atome d'halogène, un groupe alkyle en $C_1$-$C_4$, alkoxy en $C_1$-$C_4$ et trifluorométhyle; et un groupe $COR_7$,

$R_7$ étant choisi parmi un groupe alkyle en $C_1$-$C_4$, phényle, et un groupe

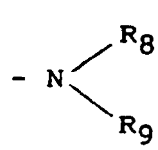 ,

$R_8$ et $R_9$ étant choisis parmi un atome d'hydrogène et un groupe alkyle en $C_1$-$C_4$,

et $R_{10}$ représente un atome d'hydrogène ou un groupe alkyle en $C_1$-$C_4$, sous réserve que $R_3$ ne représente pas un atome d'hydrogène ou un groupe trifluorométhyle, alkyle, alkoxy ou hydroxyalkyle en $C_1$-$C_4$ lorsque Y est O.

Les composés de formule (I) comprennent notamment les composés dans lesquels l'hétérocycle est un pyrazole c'est-à-dire Y représente le groupe -$NR_4$-, dans lequel $R_4$ est de préférence choisi parmi un atome d'hydrogène, le groupe méthyle, acétyle et méthoxyéthyle.

D'autres composés de formule I sont ceux dans lesquels l'hétérocycle est un isoxazole (Y est O), et parmi ceux-ci on préfère ceux dans lesquels $R_3$ représente un groupe halogénoalkyle ou acétyle.

Les composés de formule I que l'on préfère sont également ceux dans lesquels $R_1$ représente le groupe méthyle, $R_2$ représente un atome de chlore, le groupe méthyle ou isopropyle, $R_3$ représente le groupe méthyle

ou méthoxy lorsque l'hétérocycle est un pyrazole et le groupe $-CH_2OR_5$ dans lequel $R_5$ est un groupe méthyle, phényle ou phényle substitué par un atome d'halogène tel que fluor ou brome, un groupe trifluorométhyle ou deux groupes méthoxy, lorsque l'hétérocycle est un isoxazole.

Plus particulièrement parmi les composés préférés selon l'invention, on peut citer :

– le (chloro-2 méthyl-6 phénylcarbamoyl)-3 acétyl-1 méthyl-5 pyrazole,
– le N-(diméthyl-1,5 pyrazolyl-3) diméthyl-2,6 benzamide
– le (diméthyl-2,6 phénylcarbamoyl)-3 diméthyl-1,5 pyrazole,
– le (chloro-2 méthyl-6 phénylcarbamoyl)-3 diméthyl-1,5 pyrazole,
– le (diméthyl-2,6 phénylcarbamoyl)-3 méthyl-1 méthoxy-5 pyrazole, et
– le (diméthyl-2,6 phénylcarbamoyl)-3 fluorométhyl-5 isoxazole.

Ces composés portent respectivement les numéros 2, 3, 4, 5, 15 et 31 dans les tableaux.

La présente invention a également pour objet un procédé de préparation des composés de formule générale I, caractérisé en ce que l'on fait réagir un composé de formule générale

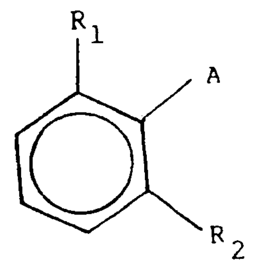

II

dans laquelle $R_1$ et $R_2$ ont les mêmes significations que dans la formule I et A représente un groupe-COOH, -COCl ou $-N(R_6)H$ dans lequel $R_6$ représente un atome d'hydrogène ou un groupe alkyle en $C_1$-$C_4$, avec un composé de formule générale

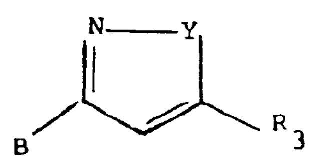

III

dans laquelle Y et $R_3$ ont les mêmes significations que dans la formule I et B représente un groupe -COOH, COCl, -CH=CH-COOH, -CH=CH-COCl ou $-N(R_6)H$, $R_6$ étant H ou un alkyle en $C_1$-$C_4$. En particulier,

a) quand Z représente les groupes $-N(R_6)$-CO-, ou -NH-CO-CH=CH-, $R_6$ étant tel que défini dans la formule générale I, on condense une amine de formule générale II dans laquelle A représente le groupe $-NR_6H$ avec un acide ou un chlorure d'acide de formule générale III dans laquelle B représente un groupe -COOH, -COCl, -CH=CH-COOH ou -CH=CH-COCl.

Quand on utilise un acide de formule générale III, on effectue la réaction en présence de dicyclohexylcarbodiimide (DCC) ou carbonyldiimidazole (CDI) dans un solvant qui peut être le diméthylforma-mide (DMF) ou le tétrahydrofuranne (THF) à une température de 10 à25°C.

Dans le cas où l'on utilise un chlorure d'acide (III), on réalise la condensation en présence d'un capteur de proton tel que la triéthylamine ou le carbonate de potassium à une température de 20 à 120°C dans un solvant neutre tel que le toluène, l'acétone, etc.

Les acides de formule générale III (Y = $-NR_4$-) sont connus de la littérature.

Il s'agit en particulier des acides :

– méthyl-1 méthoxy-5 pyrazole carboxylique-3
– (diméthyl-1,5 pyrazolyl)-3 acrylique
– diméthyl-1,5 pyrazole carboxylique-3
– méthyl-5 pyrazole carboxylique-3

Les amines de formule générale II sont toutes des produits disponibles dans le commerce ou décrits dans la littérature.

b) Quand Z représente le groupe -CO-NH-, on condense un composé de formule générale II dans laquelle A représente -COOH ou -COCl avec un composé de formule générale III dans laquelle B représente $-NH_2$. Cette réaction est effectuée de la même manière que celle sous a) ci-dessus. Les acides et chlorures d'acide de formule II et les amines de formule III sont disponibles dans le commerce ou décrits dans la littérature.

c) Les composés de formule I dans laquelle $R_4$ représente un groupe benzyle, éventuellement substitué, acyle, ou alkyle substitué par alkanoyloxy, dialkylamino, alkoxy ou phénoxy sont préparés en faisant réagir sur un composé de formule I dans laquelle $R_4$ représente un atome d'hydrogène, un composé de formule $R_4X$, $R_4$ étant tel que défini précédemment et X représentant un atome d'halogène.

d) Les composés de formule III telle que définie précédemment dans laquelle l'hétérocycle est un isoxazole (Y représentant -O-) et $R_3$ représente le groupe $CHR_{10}OR_5$, $R_5$ et $R_{10}$ étant tels que définis précédemment et B représente le groupe -COOH peuvent être préparés

– soit en faisant réagir un composé de formule

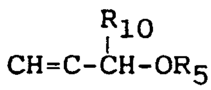

V

$R_5$ et $R_{10}$ étant tels que définis précédemment avec un composé de formule

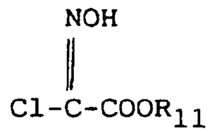

VI

$R_{11}$ représentant un groupe méthyle ou éthyle pour obtenir un composé de formule

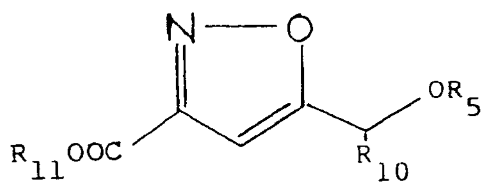

VII

$R_5$, $R_{10}$ et $R_{11}$ étant tels que définis précédemment,

– soit en faisant réagir un composé de formule

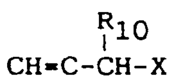

VIII

$R_{10}$ étant tel que défini précédemment et X représentant un atome de chlore ou de brome avec un composé de formule

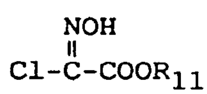

IX

$R_{11}$ étant tel que défini précédemment pour obtenir un composé de formule

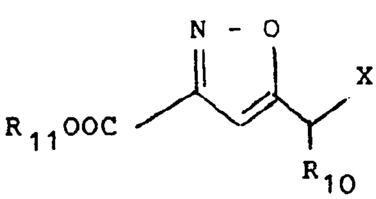

X

X étant tel que défini ci-dessus,
que l'on fait réagir ensuite avec un alcoolate de formule $R_5O$-Na, $R_5$ étant tel que défini précédemment pour obtenir un composé de formule X telle que définie ci-dessus, puis en hydrolysant l'ester de formule X pour obtenir l'acide correspondant de formule III.

Certains composés de l'invention possèdent un ou plusieurs carbones assymétriques. Les isomères optiques correspondants font aussi partie de l'invention.

Les exemples suivants illustrent la préparation des composés de formule I.

EXEMPLE 1

Préparation du (diméthyl-2,6 phénylcarbamoyl)-3 diméthyl-1,5-pyrazole (composé n° 4)

a) préparation de l'ester éthylique de l'acide (diméthyl-1,5) pyrazole carboxylique-3

Dans un ballon de 250 ml, on dilue 16,3 g (103,2 mmoles) de $CH_3$-CO-$CH_2$-CO-CO-O-$C_2H_5$. On ajoute goutte à goutte 5,5 ml (103,2 mmoles) de méthylhydrazine et on chauffe au reflux pendant 2 heures. On laisse refroidir et on évapore le solvant. On obtient les deux isomères du titre en séparant le mélange obtenu par distillation fractionnée.

On obtient :

. l'ester éthylique de l'acide (diméthyl-1,5) pyrazole carboxylique-3 : 9,85 g (rendement 56,9%). Eb : 92-96°C/0,17 mmHg.

b) préparation de l'acide diméthyl-1,5 pyrazole carboxylique-3

A 9,8 g (58,3 mmoles) de l'ester éthylique de l'acide diméthyl-1,5 pyrazole carboxylique-3 on ajoute 50 ml d'éthanol et 2,3 g (58,3 mmoles) de soude additionnés de 19,5 ml d'eau. On obtient 6,15 g d'acide diméthyl-1,5 pyrazole carboxylique-3 sous forme de cristaux blancs (rendement : 75,4%). Pf = 175°C.

c) préparation du (diméthyl-2,6 phénylcarbamoyl)-3 diméthyl-1,5 pyrazole

A 18,4 g (131,4 mmoles) du composé obtenu précédemment, on ajoute 11,3 ml (131,4 mmoles) de chlorure de thionyle dans 1 l de toluène puis 15,9 g (131,4 mmoles) de diméthyl-2,6 aniline, 18 ml (131,4 mmoles) de triéthylamine dans 750 ml de toluène. On obtient 27,65 g du produit du titre sous forme de cristaux ocres.

Rendement : 86,6%

Pf = 155°C

EXEMPLE 2

Préparation du (diméthyl-2,6 phénylcarbamoyl)-3 méthyl-5 pyrazole (composé n° 1)
(D.E. Butler et H.A. Dewald JOC 1975, 40(9), 1353))

Dans un ballon de 100 ml surmonté d'un réfrigérant muni d'une garde à $CaCl_2$, on introduit 5,8 g (40 mmoles) de chlorhydrate de pyridine et 2,4 g (10 mmoles) du composé n° 4 obtenu à l'exemple précédent. On chauffe à 220°C pendant 12 heures. On laisse refroidir, dilue à la saumure, extrait à l'acétate d'éthyle et sèche sur $MgSO_4$ anhydre. Après évaporation du solvant, purification par flash-chromatographie et recristallisation dans $CH_2Cl_2$, on obtient 0,4 g du produit du titre sous forme de cristaux de couleur beige.

Rendement = 21,2%

Pf = 198°C.

Le composé du titre peut également être obtenu en procédant de la manière suivante :

A 4 g (30 mmoles) d'acide méthyl-5 pyrazole carboxylique-3, on ajoute 7,1 g (60 mmoles) de chlorure de thionyle, 150 ml de toluène et quelques gouttes de DMF. Au produit obtenu, on ajoute alors 7,7 g de diméthyl-2,6 aniline (60 mmoles), 200 ml de toluène et encore quelques gouttes de DMF. On obtient 2,60 g du produit du titre sous forme de cristaux ocres.

Rendement : 37,8%

Pf = 190°C.

EXEMPLE 3

Préparation du (diméthyl-2,6 phénylcarbamoyl)-3 méthyl-1 méthoxy 5 pyrazole (composé n° 15)
(d'après S. Suguira; S. Ohno; O. Ohtari; K. Iraini; T. Kitamikado; H. Asai; K. Kato; J. Med. Chem. 1977, 20 p. 80)

a) préparation de l'ester éthylique de l'acide méthyl-1 méthoxy-5 pyrazole carboxylique-3

Dans un ballon de 1 litre, on introduit 31 g (182 mmoles) d'ester éthylique de l'acide méthyl-1-hydroxy-5 pyrazole-carboxylique-3, 22,9 g (182 mmoles) de sulfate de diméthyle, 12,5 g (91 mmoles) de $K_2CO_3$ et 500 ml d'acétone. On chauffe au reflux pendant 5 heures.

On laisse refroidir, filtre le précipité obtenu, évapore le solvant reprend le résidu dans de l'acétone et filtre le précipité obtenu. On obtient 12 g de cristaux blancs (Pf : 78°C). Le filtrat contient encore 19 g de produit impur (Rendement : 90%).

b) préparation de l'acide méthyl-1 méthoxy-5 pyrazole carboxylique-3

A 12 g (65,2 mmoles) de l'ester obtenu précédemment, on ajoute 100 ml d'éthanol et 2,6 g (62,5 mmoles) de soude dans 50 ml d'eau. On laisse une nuit à température ambiante. On obtient 9 g d'acide.

Rendement : 88,5%

Pf = 194°C.

c) préparation du (diméthyl-2,6 phénylcarbamoyl)-3 méthyl-1 méthoxy-5 pyrazole

A 9 g (57,6 mmoles) du produit obtenu précédemment, on ajoute 200 ml de toluène et 6,86 g (57,6 mmoles) de chlorure de thionyle. On chauffe au reflux pendant 6 heures. On obtient 10,5 g de chlorure d'acide (Pf = 76°C).

On ajoute au chlorure d'acide obtenu 7,28 g (60,1 mmoles) de diméthylaniline, 6,07 g (60,1 mmoles) de triéthylamine et 200 g de toluène. On obtient 11 g du produit du titre sous forme de cristaux blancs.

Rendement : 73,7%

Pf = 144°C.

EXEMPLE 4

Préparation du diméthyl-2,6 phénylcarbamoyl)-3 benzyl-1 méthyl-5 pyrazole (composé n° 14)

Le produit est préparé comme décrit par G. Tarrago et A. Ramdari (J. Hetero Chem. 17, 137 (1980).

Dans un ballon de 100 ml surmonté d'une colonne réfrigérante munie d'une garde à $CaCl_2$, on introduit 1 g (4,4 mmoles) du composé n° 1 obtenu à l'exemple n° 2. On ajoute 30 ml de DMF, 7,50 mg (4,4 mmoles) de bromure de benzyle et 2,1 g (13 mmoles) d'iodure de potassium. On chauffe pendant 1 h à 100°C. Après évaporation du solvant, on reprend le résidu obtenu dans du chloroforme. On lave la phase organique avec une solution de thiosulfate de sodium puis avec de l'eau et on sèche sur $Na_2SO_4$.

Après évaporation du solvant et purification par flash-chromatograhie, on obtient 0,2 g du produit du titre sous forme de cristaux blancs.

Rendement : 14,3%

Pf = 147°C

EXEMPLE 5

Préparation du (diméthyl-2,6 phénylcarbamoyl)-3 méthoxyméthyl-5 isoxazole (composé n° 21)

a) préparation de l'ester éthylique de l'acide méthoxyméthyl-5 isoxazole carboxylique-3

9 g (0,128 mole) d'éther méthoxypropargylique préparé selon L.A. CABE, D.R. BENEDICT, T.A. BIANCHI (Synthesis, 428, 1979) sont dilués dans 100 ml de chloroforme. 69 g de $K_2CO_3$ (0,5 mole) sont ajoutés à température ambiante et le mélange obtenu agité mécaniquement à cette température.

19,5 g (0,129 mole) de chlorooximidoacétate d'éthyle sont ensuite ajoutés goutte à goutte (J. ORG. CHEM., 48(3), 371, 1983).

La réaction achevée, le milieu réactionnel est rincé avec 50 ml de $CHCl_3$ et agité pendant 48 heures. Le $K_2CO_3$ est filtré, le chloroforme évaporé et le produit purifié par distillation. On obtient 13 g de produit pur.

Rendement : 59%

$Eb_{20}$ = 168-172°C

b) préparation de l'acide méthoxyméthyl-5 isoxazole carboxylique-3

13 g (0,075 mole) du produit obtenu à l'étape précédente sont mis en suspension dans 40 ml d'eau. Le mélange est refroidi dans un bain de glace. 12 ml de soude à 30% (0,120 mole) sont alors ajoutés. On laisse revenir à température ambiante et on agite pendant 1 heure. On refroidit à nouveau le milieu réactionnel et on ajoute 12 ml d'HCl à 37%. On évapore l'eau, reprend le résidu par de l'acétone, filtre, sèche sur $MgSO_4$ et évapore le solvant.

c) préparation du (diméthyl-2,6 phénylcarbamoyl)-3 méthoxyméthyl-5 isoxazole

On procède comme décrit dans J. MED. CHEM., 30(11), 2008-2012.

4,3 g de 2,6 diméthylaniline (0,036 mole) sont dilués dans 100 ml de THF et refroidis dans un bain d'eau et de glace. On ajoute 4,2 ml de $POCl_3$ (0,045 mole), puis 7 g (0,045 mole) du produit obtenu à l'étape précédente. On laisse le mélange en contact pendant 1/2 heure. On ajoute lentement 9 ml de triéthylamine dans 50 ml de THF. On laisse revenir lentement à température ambiante et on agite pendant 1 nuit.

On extrait à l'acétate d'éthyle, lave avec de l'eau saturée en NaCl jusqu'à pH neutre, sèche sur $MgSO_4$ et évapore le solvant. Le produit brut obtenu est purifié sur une colonne de silice (éluant $CH_2Cl_2$). On obtient 4,3 g du produit du titre.

Rendement : 46%.

## EXEMPLE 6

Préparation du (diméthyl-2,6 phénylcarbamoyl)-3 (diméthoxy-2,6 phénoxyméthyl)-5 isoxazole (composé n° 25)

a) préparation de l'ester méthylique de l'acide (diméthoxy-2,6 phénoxyméthyl)-5 isoxazole carboxylique-3

On chauffe au reflux pendant 6 heures 5,1 g (0,023 mole) d'ester méthylique de l'acide bromométhyl-5 isoxazole carboxylique-3, 3,6 g de diméthoxy-2,6 phénol (0,023 mole) et 5 g de $K_2CO_3$ (0,036 mole) dans 150 ml d'acétone.

On filtre le $K_2CO_3$ rince avec de l'acétone et évapore le solvant.

On obtient 6,2 g de produit pur (rendement : 100%).

b) préparation de l'acide (diméthoxy-2,6 phénoxyméthyl)-5 isoxazole carboxylique-3

On dissout 6,7 g de l'ester obtenu à l'étape précédente dans 200 ml de méthanol. On ajoute 1,5 g de soude (0,0375 mole) dans 20 ml d'eau et on agite à température ambiante jusqu'à disparition de toute trace de l'ester. On évapore ensuite le solvant. On dissout le sel de sodium dans de l'eau, acidifie à froid jusqu'à pH2 pour obtenir la précipitation de l'acide. On filtre et sèche sur $P_2O_5$. On obtient ainsi 6,5 g de produit du titre.

Rendement : 100%.

c) Préparation du (diméthyl-2,6 phénylcarbamoyl)-3 (diméthoxy-2,6 phénoxyméthyl)-5 isoxazole

On dilue dans 150 ml de THF, 6,5 g du produit obtenu à l'étape précédente et 2,9 g de diméthyl-2,6 aniline (0,023 mole) et on refroidit à 0°C.

On ajoute 2,8 ml de $POCl_3$ (0,030 mole). On laisse en contact pendant 1/2 heure. On ajoute ensuite lentement 7 ml de triéthylamine (0,070 mole) dans 50 ml de THF. On laisse en contact pendant 12 heures, filtre sur verre fritté et évapore le solvant.

On purifie le produit brut obtenu sur une colonne de silice (éluant : $CH_2Cl_2$).

On obtient 4,6 g de produit.

Rendement : 50%.

## EXEMPLE 7

Préparation du diméthyl-2,6 phénylcarbamoyl)-3 acétoxyméthyl-5 isoxazole (composé n° 27)

a) préparation de l'ester méthylique de l'acide hydroxyméthyl-5 isoxazole carboxylique-3

60 g de chlorooximidoacétate de méthyle (0,436 mole) dilué dans 100 ml de chloroforme sont ajoutés très lentement à une solution composée de 30 g d'alcool propargylique (0,535 mole) et 180 g de $K_2CO_3$ (1,3 mole) dans 500 ml de chloroforme.

L'addition s'accompagne d'un échauffement produisant un reflux du chloroforme.

Après retour à la température ambiante, le mélange est agité pendant 6 heures.

Une chromatographie sur couche mince (éluant: $CH_2Cl_2$/MeOH : 98/2) montre un taux de conversion de 100%.

Le mélange réactionnel est filtré sur verre fritté, le résidu rincé au chloroforme et le solvant évaporé sous pression réduite.

On obtient le produit du titre brut avec une pureté d'environ 100%.

b) préparation de l'acide hydroxyméthyl-5 isoxazole carboxylique-3

38 g du produit obtenu à l'étape précédente (0,242 mole) sont mis en suspension dans 50 ml d'eau distillée.

Après refroidissement sur bain de glace, 10 g de soude (0,25 mole) dans 50 ml d'eau distillée sont ajoutés goutte à goutte.

Le mélange réactionnel est agité à 0°C pendant 1 heure, puis pendant 1 heure à température ambiante.

On refroidit à 0°C, neutralise la soude en excès par de l'HCl dilué et acidifie jusqu'à pH2.

On évapore l'eau, reprend par de l'acétone, filtre le NaCl insoluble, sèche sur $MgSO_4$ et évapore le solvant.

c) préparation de l'acide acétoxyméthyl-5 isoxazole carboxylique-3

25 g du produit obtenu à l'étape précédente (0,16 mole) sont mis en suspension dans un mélange composé de 125 ml d'anhydride acétique et 25 ml d'acide acétique pur.

5 gouttes d'$H_2SO_4$ sont ajoutés, et après dissolution totale des matières premières, on chauffe le mélange réactionnel pendant 1 heure à 60°C.

Une chromatographie sur couche mince (éluant: $CH_2Cl_2$/MeOH : 80/20) montre qu'à ce stade, la réaction est totale.

L'acide acétique et l'anhydride acétique sont éliminés par distillation sous pression réduite.

On obtient 29 g du produit du titre.

Rendement : 98%

Pf = 84°C.

d) préparation du (diméthyl-2,6 phénylcarbamoyl)-3 acétoxyméthyl-5 isoxazole

20 g de diméthyl-2,6 aniline (0,16 mole) sont dilués dans 200 ml de THF anhydre et refroidis à 0°C sur un bain de glace.

18,5 ml de $POCl_3$ (0,20 mole) sont ajoutés lentement puis 29 g du composé obtenu à l'étape précédente (0,16 mole) dilués dans 150 ml de THF sec.

50 ml de triéthylamine dilués dans 50 ml de THF sec sont ensuite additionnés très lentement.

Le mélange est agité progressivement à température ambiante pendant 12 heures.

Le chlorhydrate de triéthylamine est filtré et le mélange réactionnel lavé par de l'acétone.

Après évaporation des solvants, le produit brut obtenu est purifié sur colonne de silice (éluant: $CH_2Cl_2$).

On obtient 46 g du produit du titre.

Rendement : 100%.

EXEMPLE 8

Préparation du (diméthyl-2,6 phénylcarbamoyl)-3 t-butyryloxyméthyl-5 isoxazole (composé n° 28)

a) préparation du (diméthyl-2,6 phénylcarbamoyl)-3 hydroxyméthyl-5 isoxazole
(d'après J. Med. Chem. , 32(8), 1868, 1989)

14 g du composé obtenu à l'exemple précédent (0,0486 mole) sont dissous dans 50 ml de méthanol et refroidis à 5°C sur un bain d'eau et de glace.

4,5 ml d'ammoniaque à 28% sont ajoutés et le mélange agité à température ambiante pendant 12 heures.

Après évaporation du solvant, le produit brut est purifié sur colonne de silice (éluant : gradient $CH_2Cl_2$-$CH_2Cl_2$/MeOH : 97/3).

On obtient 9 g du produit du titre pur.

Rendement : 76%

Pf = 100°C.

b) préparation du (diméthyl-2,6 phénylcarbamoyl)-3 t-butyryloxyméthyl-5 isoxazole

On dissout 5 g (0,020 mole) du produit obtenu à l'étape précédente dans 150 ml de THF et on refroidit dans un bain de glace et d'eau.

On ajoute 7 ml de triéthylamine (0,070 mole) en une fois, puis goutte à goutte sous vive agitation 2,6 ml de chlorure de pivaloyle (0,020 mole) dans 50 ml de THF. On laisse revenir à température ambiante, on agite pendant 12 heures, filtre sur verre fritté, évapore le solvant et purifie le produit brut obtenu sur une colonne de silice (éluant : acétone).

On obtient 5,5 g de produit du titre.

Rendement : 85%.

Les composés de ces exemples, ainsi que d'autres composés de formule I sont rassemblés dans le tableau II, plus loin.

Les composés selon l'invention se sont révélés doués de propriétés intéressantes sur le système nerveux central, en particulier de propriétés anticonvulsivantes susceptibles de les rendre utiles dans le traitement de l'épilepsie ou comme complément de la thérapeutique anticomitiale, de propriétés de protection cérébrale et d'augmentation de la mémoire.

Ainsi, l'invention comprend aussi les compositions thérapeutiques contenant à titre de principe actif les composés de formule générale I.

On donnera ci-après des résultats pharmacologiques et toxicologiques mettant en évidence les propriétés des composés de formule I.

1. Activité pharmacologique

L'activité anticonvulsivante est mesurée par les tests à l'électrochoc et au pentétrazole selon la technique de A. SWINYARD (ADD PROGRAM OF NINCDS BY H.J. KUPFERBERG. E.A. SWINYARD AND G.D. GLADDING in advances in epileptology/XIIth Epilepsy International Symposium edited by M. DAM, L. GRAM and J.K. PENRYRAVEN press NEW-YORK 1981). Les composés sont toujours administrés (au 1/10e de la DL50) par injections IP à des souris SWISS CD1 (Charles River) d'un poids moyen de 20-25g. Tous les produits sont dissous dans une solution à 0,9% de chlorure de sodium ou mis en suspension dans une solution de carboxyméthyl-cellulose ou de tween à 1%.

Test à l'électrochoc. Des lots de 10 souris (1 lot témoin et un lot traité) sont utilisés pour chaque composé. Le lot traité reçoit le produit à tester par voie intrapéritonéale, d'une part 30mn avant l'électrochoc et d'autre part 4 heures avant l'électrochoc. Celui-ci est appliqué à l'aide d'électrodes cornéennes (50 milliampères pendant 0,2 seconde). La protection est mesurée par le pourcentage d'animaux neprésentant pas d'extension des pattes postérieures.

Crises au pentétrazole. On injecte par voie sous-cutanée à des lots de 10 souris (1 lot témoin et un lot traité) 70 mg/kg de pentétrazole à raison de 0.2 ml/20g de poids corporel. Les produits à tester sont administrés d'une part 30 mn avant le pentétrazole, d'autre part 4 heures avant le pentétrazole, par voie intrapéritonéale. Les animaux sont observés pendant 30 mn. On note le nombre de crises cloniques d'une durée supérieure ou égale à 5 secondes et le pourcentage des animaux protégés contre les crises cloniques.

Les résultats sont consignés dans le tableau ci-dessous.

2. Activité protectrice cérébrale

Cinq souris (20 à 25g) reçoivent une administration par voie intrapéritonéale de produit ou de liquide-véhicule 30 min avant d'être placées dans une enceinte close, où la pression atmosphérique est diminuée de 210 mm Hg. La durée de survie (en secondes) est mesurée depuis l'induction de l'hypoxie jusqu'à la disparition des mouvements respiratoires.

3. Détermination de la dose léthale 50

La toxicité est mesurée par la technique de MILLER et TAINTER par voie intrapéritonéale. Les résultats sont consignés dans le tableau I ci-dessous.

TABLEAU I

| Composés | DL.50 mg/kg | % protection pour Electrochoc 30 min. | Electrochoc 4h | Pentétrazole 30 mn. | Pentétrazole 4h | hypoxie induite par l'altitude augmentation temps de survie en % par rapport au témoin |
|---|---|---|---|---|---|---|
| 2 | 350 | 100 | 0 | 80 | 0 | |
| 3 | 1000 | 100 | 0 | 50 | 0 | |
| 4 | 150 | 100 | 0 | 30 | 10 | 60 |
| 5 | 500 | 100 | 10 | 40 | 10 | |
| 7 | 400 | 70 | 0 | 30 | 0 | |
| 15 | 400 | 10 | 0 | 0 | 10 | 21 |
| 29 | 2000 | 70 | 0 | 10 | 10 | |
| 31 | 375 | 100 | 0 | 0 | 0 | |
| 41 | 350 | 100 | 0 | 50 | 0 | |

Les compositions thérapeutiques selon l'invention peuvent être administrées par voie orale, parentérale ou endorectale.

Elles peuvent être sous la forme de comprimés, dragées, gélules, solutions ou suspensions injectables et suppositoires.

La quantité de principe actif administrée dépend évidemment du patient qui est traité, de la voie d'administration et de la sévérité de la maladie.

Cependant, la posologie quotidienne sera de l'ordre de 10 à 300 mg.

La dose unitaire peut être de 10 à 100 mg.

EXEMPLES DE FORMULATION

1) Formule type comprimé :

| | | |
|---|---|---|
| Pour 5000 comprimés à 20 mg | | |
| Composé de l'exemple 2 | : | 100g |
| Cellulose microcristalline | : | 1000g |
| Carboxyméthyl cellulose sodique | : | 15g |
| Stéarate de magnésium | : | 10g |
| Total | = | 1125g |

– Mélanger tous les constituants dans un mélangeur Turbula$^R$ pendant 10min
– Comprimer sur machine alternative, poids théorique : 225 mg.

2) Formules type gélules :

| | | |
|---|---|---|
| Pour 5000 gélules taille 1 dosées à 10 mg | | |
| Composé de l'exemple 2 | : | 50g |
| Amidon de maïs | : | 150g |
| Lactose | : | 1250g |
| PVP K30 | : | 75g |
| Talc | : | 30g |
| Stéarate de magnésium | : | 10g |
| Total | = | 1565g |
| Alcool à 50° | = | QS |

– Dans un mélangeur planétaire, mélanger pendant 10 min les constituants suivants : composé N° 1 - amidon de maïs - lactose - PVP.
– Toujours sous agitation, verser lentement l'alcool jusqu'à granulation satisfaisante.
– Etendre sur plateaux, faire sécher en étuve à 50°C.
– Calibrer sur granulateur oscillant, grille de 1 mm.
– Mélanger le grain avec le talc et le stéarate de magnésium 10 min au Turbula$^R$.
– Mettre en gélule, poids théorique : 313 mg.

TABLEAU II : Exemples de composés de l'invention

| N° composé | 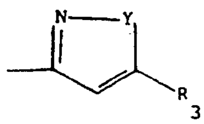 | Z | $R_1$ | $R_2$ | P.F. (°C) |
|---|---|---|---|---|---|
| 1 | 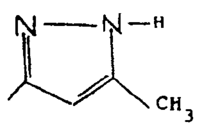 | -NH-CO- | $CH_3$ | $CH_3$ | 190 |
| 2 | 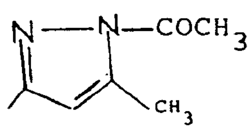 | -NH-CO- | $CH_3$ | Cl | 151 |
| 3 | 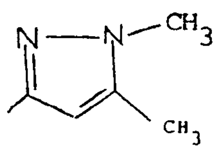 | -CO-NH- | $CH_3$ | $CH_3$ | 186 |
| 4 | 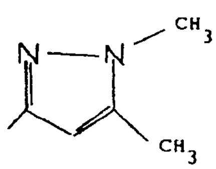 | -NH-CO- | $CH_3$ | $CH_3$ | 144 |

| | | | | | |
|---|---|---|---|---|---|
| 5 | 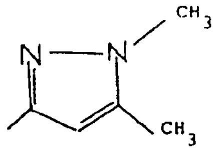 | -NH-CO- | $CH_3$ | Cl | 182 |
| 6 | 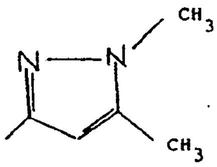 | -NH-CO- | $C_3H_7$-iso | $CH_3$ | 100 |
| 7 | 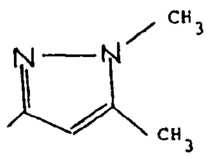 | $CH_3$ -N-CO- | $CH_3$ | $CH_3$ | 134 |
| 8 | 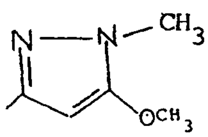 | -CO-NH- | $CH_3$ | $CH_3$ | 157 |
| 9 | 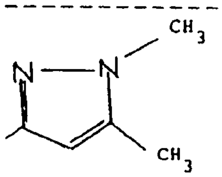 | -NH-CO-CH=CH- | $CH_3$ | $CH_3$ | 112 |
| 10 | 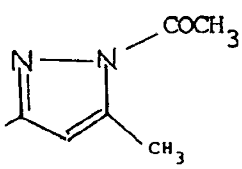 | -CO-NH- | $CH_3$ | $CH_3$ | 182 |
| 11 | 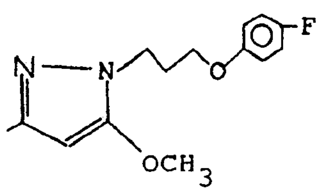 | -NH-CO- | $CH_3$ | Cl | 108 |

| | | | | | |
|---|---|---|---|---|---|
| 14 | 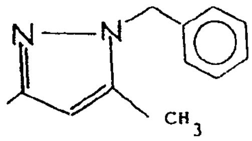 | -NH-CO | $CH_3$ | $CH_3$ | 147 |
| 15 | 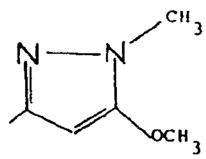 | -NH-CO- | $CH_3$ | $CH_3$ | 134 |
| 16 | 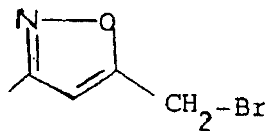 | -NH-CO- | $CH_3$ | $CH_3$ | 142 |
| 18 | 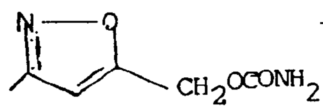 | -NH-CO- | $CH_3$ | $CH_3$ | 135 |
| 20 | 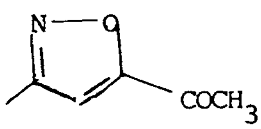 | -NH-CO- | $CH_3$ | $CH_3$ | 117 |
| 21 | 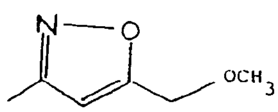 | -NH-CO- | $CH_3$ | $CH_3$ | 87 |
| 22 | 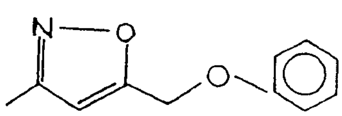 | -NH-CO- | $CH_3$ | $CH_3$ | 116 |
| 23 | 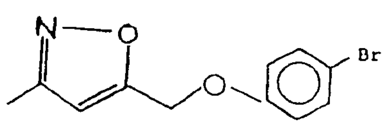 | -NH-CO- | $CH_3$ | $CH_3$ | 99 |

| | | | | | |
|---|---|---|---|---|---|
| 24 | 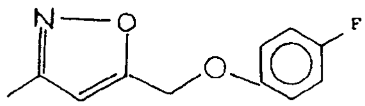 | -NH-CO- | $CH_3$ | $CH_3$ | 107 |
| 25 | 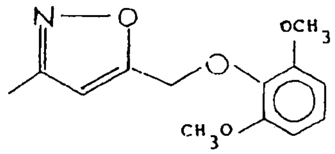 | -NH-CO- | $CH_3$ | $CH_3$ | 75 |
| 26 | 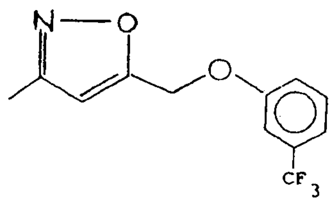 | -NH-CO- | $CH_3$ | $CH_3$ | 118 |
| 27 | 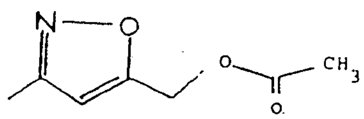 | -NH-CO- | $CH_3$ | $CH_3$ | 71 |
| 28 | 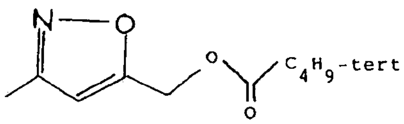 | -NH-CO- | $CH_3$ | $CH_3$ | 132 |
| 29 | 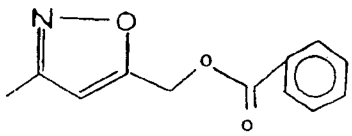 | -NH-CO- | $CH_3$ | $CH_3$ | 69 |
| 30 | 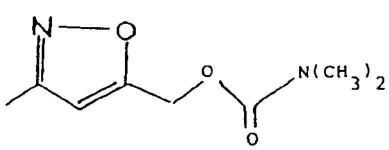 | -NH-CO- | $CH_3$ | $CH_3$ | 82 |

| | | | | | |
|---|---|---|---|---|---|
| 31 | 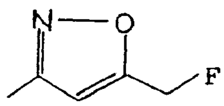 | -NH-CO- | $CH_3$ | $CH_3$ | 90 |
| 32 | 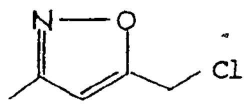 | -NH-CO- | $CH_3$ | $CH_3$ | 130 |
| 33 | 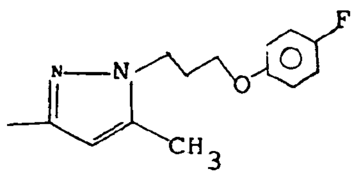 | -NH-CO- | $CH_3$ | Cl | 157 |
| 34 | 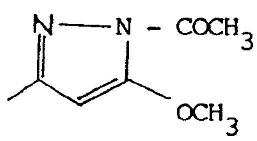 | -NH-CO- | $CH_3$ | Cl | 192 |
| 35 | 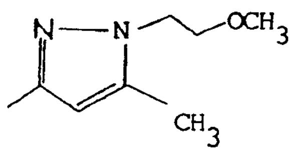 | -NH-CO- | $CH_3$ | Cl | 97 |
| 36 | 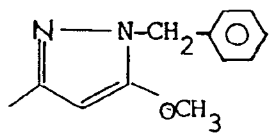 | -NH-CO- | $CH_3$ | Cl | 97 |
| 37 | 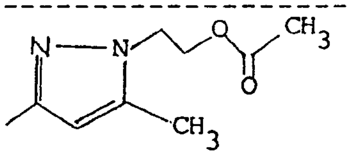 | -NH-CO- | $CH_3$ | Cl | 113 |

| | | | | | |
|---|---|---|---|---|---|
| 38 | 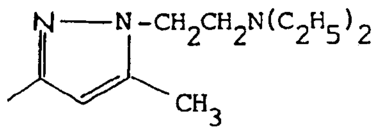 | -NH-CO- | $CH_3$ | Cl | 66 |
| 39 | 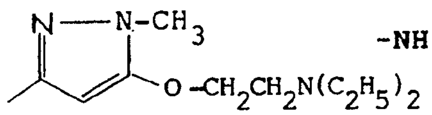 | -NH-CO- | $CH_3$ | Cl | 71 |
| 40 | 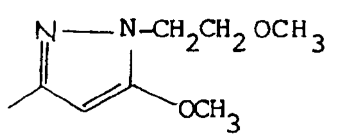 | -NH-CO- | $CH_3$ | Cl | 97 |
| 41 | 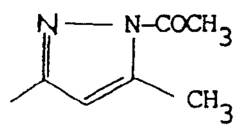 | -NH-CO- | $CH_3$ | $CH_3$ | 181 |

**Revendications**

1. Composé hétérocycliques de formule générale (I)

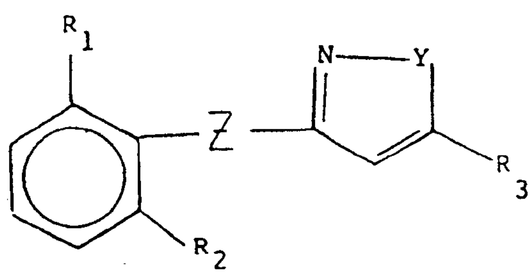

I

dans laquelle
Y est choisi parmi -O-, -S- et

R4
|
-N-,

$R_4$ étant choisi parmi un atome d'hydrogène, un groupe alkyle en $C_1$-$C_4$, benzyle, halogénobenzyle, acyle en $C_2$-$C_7$ et un groupe alkyle en $C_1$-$C_4$ substitué par un groupe alkanoyl($C_2$-$C_7$)oxy, dialkyl($C_1$-$C_4$)amino, alkoxy en $C_1$-$C_4$, phénoxy, ou halogénophénoxy,
Z est choisi parmi les groupes -CO-N($R_6$)-, -NH-CO-CH=CH- et -N($R_6$)-CO- dans lesquels $R_6$ est choisi parmi un atome d'hydrogène et un groupe alkyle en $C_1$-$C_4$,
$R_1$ est un groupe alkyle en $C_1$-$C_4$,
$R_2$ est choisi parmi un groupe alkyle en $C_1$-$C_4$, et un atome d'halogène,
$R_3$ est choisi parmi un atome d'hydrogène, un groupe alkyle en $C_1$-$C_4$, alkoxy en $C_1$-$C_4$, hydroxyalkyle en $C_1$-$C_4$,

halogénoalkyle en $C_1$-$C_4$, alkanoyle en $C_2$-$C_7$ et un groupe $-CHR_{10}OR_5$ dans lequel $R_5$ est choisi parmi un groupe alkyle en $C_1$-$C_4$, un groupe phényle non substitué ou substitué par un ou plusieurs substituants choisis parmi un atome d'halogène, un groupe alkyle en $C_1$-$C_4$, alkoxy en $C_1$-$C_4$ et trifluorométhyle; et un groupe $COR_7$, $R_7$ étant choisi parmi un groupe alkyle en $C_1$-$C_4$, phényle, et un groupe

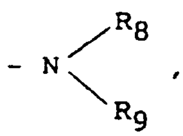

$R_8$ et $R_9$ étant choisis parmi un atome d'hydrogène et un groupe alkyle en $C_1$-$C_4$,
et $R_{10}$ représente un atome d'hydrogène ou un groupe alkyle en $C_1$-$C_4$, sous réserve que $R_3$ ne représente pas un atome d'hydrogène ou un groupe trifluorométhyle, alkyle, alkoxy ou hydroxyalkyle en $C_1$-$C_4$ lorsque Y est O.

**2** - Composés hétérocycliques selon la revendication 1, caractérisés en ce que Y est un groupe $-NR_4-$.

**3** - Composés hétérocycliques selon la revendication 1, caractérisés en ce que l'hétérocyclique est un groupe isoxazole de formule

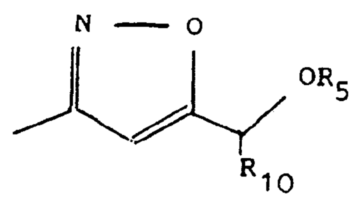

$R_5$ et $R_{10}$ étant tels que définis à la revendication 1.

**4** - Composés hétérocycliques selon l'une quelconque des revendications précédentes, caractérisés en ce que Z représente le groupe $-N(R_6)-CO-$ dans lequel $R_6$ représente un atome d'hydrogène ou le groupe méthyle.

**5** - Composés hétérocycliques selon l'une quelconque des revendications précédentes, caractérisés en ce que $R_1$ et $R_2$ représentent chacun le groupe méthyle.

**6** - Composés hétérocycliques selon la revendication 1, choisis parmi :
- le (chloro-2 méthyl-6 phénylcarbamoyl)-3 acétyl-1 méthyl-5 pyrazole,
- le N-(diméthyl-1,5 pyrazolyl-3) diméthyl-2,6 benzamide
- le (diméthyl-2,6 phénylcarbamoyl)-3 diméthyl-1,5 pyrazole,
- le (chloro-2 méthyl-6 phénylcarbamoyl)-3 diméthyl-1,5 pyrazole,
- le (diméthyl-2,6 phénylcarbamoyl)-3 méthyl-1 méthoxy-5 pyrazole, et
- le (diméthyl-2,6 phénylcarbamoyl)-3 fluorométhyl-5 isoxazole.

**7** - Procédé de préparation d'un composé selon la revendication 1, caractérisé en ce que l'on fait réagir un composé de formule générale

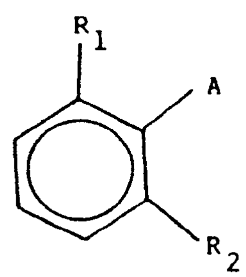

II

dans laquelle $R_1$ et $R_2$ ont les mêmes significations que dans la formule I et A représente un groupe -COOH, -COCl ou $-N(R_6)H$ dans lequel $R_6$ représente un atome d'hydrogène ou un groupe alkyle en $C_1$-$C_4$, avec un composé de formule générale

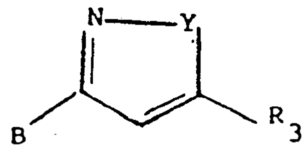

III

dans laquelle les symboles Y et $R_3$ ont la même signification que dans la formule I et
B représente un groupement -COOH, -CH=CH-COOH, -CH=CH-COCl ou $-NR_6H$, dans lequel $R_6$ représente un atome d'hydrogène ou un groupe alkyle en $C_1$-$C_4$.

**8** - Procédé de préparation des composés de formule I dans laquelle Z représente les groupes $-N(R_6)-CO$, selon la revendication 7, caractérisé en ce que l'on condense une amine de formule générale II dans laquelle A représente le groupe $-NR_6H$ avec un acide ou un chlorure d'acide de formule générale III dans laquelle B représente un groupe -COOH, -COCl -CH=CH-COOH ou -CH=CH-COCl.

**9** - Composition pharmaceutique, caractérisée en ce qu'elle contient à titre de principe actif un composé selon l'une quelconque des revendications 1 à 6.

**Revendications pour l'Etat contractant suivant : ES**

**1.** Procédé de préparation d'un composé hétérocycliques de formule générale **(I)**

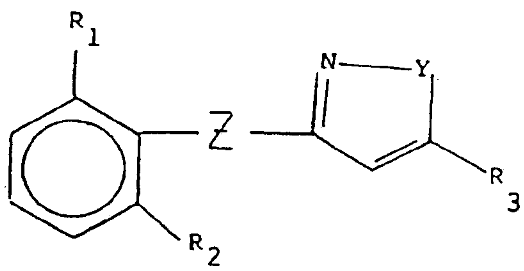

I

dans laquelle
Y est choisi parmi -O-, -S- et

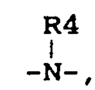

$R_4$ étant choisi parmi un atome d'hydrogène, un groupe alkyle en $C_1$-$C_4$, benzyle, halogénobenzyle, acyle en $C_2$-$C_7$ et un groupe alkyle en $C_1$-$C_4$ substitué par un groupe alkanoyl($C_2$-$C_7$)oxy, dialkyl($C_1$-$C_4$)amino, alkoxy en $C_1$-$C_4$, phénoxy, ou halogénophénoxy,
Z est choisi parmi les groupes $-CO-N(R_6)-$, -NH-CO-CH=CH- et $-N(R_6)-CO-$ dans lesquels $R_6$ est choisi parmi un atome d'hydrogène et un groupe alkyle en $C_1$-$C_4$,
$R_1$ est un groupe alkyle en $C_1$-$C_4$,
$R_2$ est choisi parmi un groupe alkyle en $C_1$-$C_4$, et un atome d'halogène,
$R_3$ est choisi parmi un atome d'hydrogène, un groupe alkyle en $C_1$-$C_4$, alkoxy en $C_1$-$C_4$, hydroxyalkyle en $C_1$-$C_4$, halogénoalkyle en $C_1$-$C_4$, alkanoyle en $C_2$-$C_7$ et un groupe $-CHR_{10}OR_5$ dans lequel $R_5$ est choisi parmi un groupe alkyle en $C_1$-$C_4$, un groupe phényle non substitué ou substitué par un ou plusieurs substituants choisis parmi un atome d'halogène, un groupe alkyle en $C_1$-$C_4$, alkoxy en $C_1$-$C_4$ et trifluorométhyle; et un groupe $COR_7$, $R_7$ étant choisi parmi un groupe alkyle en $C_1$-$C_4$, phényle, et un groupe

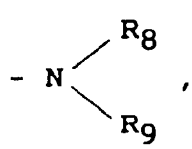

$R_8$ et $R_9$ étant choisis parmi un atome d'hydrogène et un groupe alkyle en $C_1$-$C_4$,
et $R_{10}$ représente un atome d'hydrogène ou un groupe alkyle en $C_1$-$C_4$, sous réserve que $R_3$ ne représente pas

un atome d'hydrogène ou un groupe trifluorométhyle, alkyle, alkoxy ou hydroxyalkyle en $C_1$-$C_4$ lorsque Y est O, caractérisé en ce que l'on fait réagir un composé de formule générale

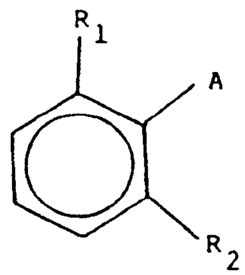

II

dans laquelle $R_1$ et $R_2$ ont les mêmes significations que dans la formule I et A représente un groupe -COOH, -COCl ou -N($R_6$)H dans lequel $R_6$ représente un atome d'hydrogène ou un groupe alkyle en $C_1$-$C_4$, avec un composé de formule générale

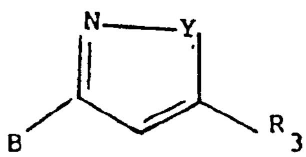

III

dans laquelle les symboles Y et $R_3$ ont la même signification que dans la formule I et B représente un groupement -COOH, -CH=CH-COOH, -CH=CH-COCl ou -$NR_6$H, dans lequel $R_6$ représente un atome d'hydrogène ou un groupe alkyle en $C_1$-$C_4$.

**2** - Procédé selon la revendication 1, caractérisé en ce que l'on prépare un composé dans lequel Y est un groupe -$NR_4$-.

**3** - Procédé selon la revendication 1, caractérisé en ce que l'on prépare un composé dans lequel l'hétérocyclique est un groupe isoxazole de formule

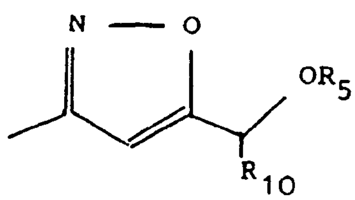

$R_5$ et $R_{10}$ étant tels que définis à la revendication 1.

**4** - Procédé selon l'une quelconque des revendications précédentes, caractérisé en ce que l'on prépare un composé dans lequel Z représente le groupe -N($R_6$)-CO- dans lequel $R_6$ représente un atome d'hydrogène ou le groupe méthyle.

**5** - Procédé selon l'une quelconque des revendications précédentes, caractérisé en ce que l'on prépare un composé dans lequel $R_1$ et $R_2$ représentent chacun le groupe méthyle.

**6** - Procédé selon la revendication 1, caractérisé en ce que l'on prépare un composé choisi parmi:

- le (chloro-2 méthyl-6 phénylcarbamoyl)-3 acétyl-1 méthyl-5 pyrazole,
- le N-(diméthyl-1,5 pyrazolyl-3) diméthyl-2,6 benzamide,
- le (diméthyl-2,6 phénylcarbamoyl)-3 diméthyl-1,5 pyrazole,
- le (chloro-2 méthyl-6 phénylcarbamoyl)-3 diméthyl-1,5 pyrazole,
- le (diméthyl-2,6 phénylcarbamoyl)-3 méthyl-1 méthoxy-5 pyrazole, et
- le (diméthyl-2,6 phénylcarbamoyl)-3 fluorométhyl-5 isoxazole.

**7** - Procédé de préparation des composés de formule I dans laquelle Z représente les groupes -N($R_6$)-CO, selon la revendication 1, caractérisé en ce que l'on condense une amine de formule générale II dans laquelle A représente le groupe -$NR_6$H avec un acide ou un chlorure d'acide de formule générale III dans laquelle B représente un groupe -COOH, -COCl -CH=CH-COOH ou -CH=CH-COCl.

**8** - Procédé de préparation d'une composition pharmaceutique, caractérisé en ce qu'on met sous forme pharmaceutiquement acceptable un composé obtenu selon l'une quelconque des revendications 1 à 7.

Office européen des brevets

# RAPPORT DE RECHERCHE EUROPEENNE

Numero de la demande

EP 91 40 1366

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | FR-A-2 313 046 (LILLY INDUSTRIES LTD) * Page 1, ligne 23 - page 3, ligne 18; page 3, lignes 5-9,14-30 * --- | 1-9 | C 07 D 231/14<br>A 61 K 31/415<br>C 07 D 231/22<br>C 07 D 261/18<br>C 07 D 271/10<br>C 07 D 285/12<br>C 07 D 231/40 |
| X | EP-A-0 049 071 (ELI LILLY AND CO.) * Pages 1-7 * --- | 1-5 | |
| A | EP-A-0 048 162 (ELI LILLY AND CO.) --- | | |
| A | US-A-3 905 997 (H. ZINNES et al.) --- | | |
| A | EP-A-0 013 376 (HOECHST AG) --- | | |
| A | EP-A-0 029 363 (MORISHITA PHARMACEUTICAL CO., LTD) --- | | |
| A | FR-A-2 315 923 (KYORIN PHARMACEUTICAL CO., LTD) --- | | |
| A | CHEMICAL ABSTRACTS, vol. 84, no. 23, 7 juin 1976, page 468, colonne 1, résumé no. 164767r, Columbus, Ohio, US; & JP-A-75 129 554 (MARUKO PHARMACEUTICAL CO., LTD) 30-03-1974 * Abrégé * --- | | **DOMAINES TECHNIQUES RECHERCHES (Int. Cl.5)** |
| P,A | EP-A-0 371 876 (NOVAPHARME) ----- | | C 07 D 231/00<br>A 61 K 31/00<br>C 07 D 261/00<br>C 07 D 271/00<br>C 07 D 285/00 |

Le présent rapport a été établi pour toutes les revendications

| Lieu de la recherche | Date d'achèvement de la recherche | Examinateur |
|---|---|---|
| LA HAYE | 04-09-1991 | DE BUYSER I.A.F. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)